# EUROPEAN PATENT SPECIFICATION

(11) **EP 1 991 285 B1**
(45) Date of publication and mention of the grant of the patent: **28.11.2012**
(21) Application number: 07749170.2
(22) Date of filing: 25.01.2007
(51) Int. Cl.: A61M 25/10

(54) **BALLOON CATHETER HAVING NANOTUBES**
BALLONKATHETER MIT NANORÖHRCHEN
CATHÉTER BALLON À NANOTUBES

(30) Priority: 10.02.2006 US 350922
(43) Date of publication of application: 19.11.2008
(73) Proprietor: Boston Scientific Limited, Christ Church (BB)
(72) Inventor: MCMORROW, David, Fort Lorenzo, Galway City (IE)
(74) Representative: Lang, Johannes
(86) International application number: PCT/US2007/001901
(87) International publication number: WO 2007/094933

(56) References cited:
- WO-A-2005/115496
- WO-A-2006/052538
- US-A1- 2005 043 787
- US-A1- 2005 096 509
- US-A1- 2005 096 731

## Description

### TECHNICAL FIELD

The present invention relates to medical devices comprising nanotubes. A medical device according to the precharacterizing portion of claim 1 or 2 is known from US 2005/0096509A.

### BACKGROUND

Stents are implantable medical devices used to maintain the diameter of a vessel after the vessel has been opened or a blockage removed. For example, a stent may be placed in a coronary artery after an angioplasty procedure is performed. Stenting is a growing field of treatment and research in medicine, and various types of stents have found use in a wide range of treatments.

In many applications, it is desirable for implanted stents to become covered in endothelial cells as early as possible after implantation of the stent. This may be particularly true with respect to arterial stenting, and especially coronary arterial stenting. Implanted stents that have not re-endothelialized (i.e., become covered to some degree with endothelial cells) are associated with adverse clinical events such as stent thrombosis. After a stent is implanted.it may take several weeks for endothelial cells to propagate from healthy areas within the vessel to the region of the implanted stent and cover the stent.

Stents may be covered with various therapeutic agents to aid acceptance of the stent or to serve other therapeutic goals. For example, stents may be covered with drugs that act to inhibit restenosis (re-blocking) of a vessel. However, it is not always feasible to put desired coatings on stents. It may be desirable, for example, to coat the stent with an agent that would encourage re-endothelialization. Unfortunately many such substances may not be capable of surviving the treatment, packaging, and sterilization that must be performed on stents prior to delivery or insertion.

US 2005/0043787 A1 discloses medical devices with coatings to promote adherence of endothelial cells. WO 2005/115496A discloses medical devices with multilayers, including layers of nanoparticles and charged polyelectrolyte layers.

### SUMMARY OF THE INVENTION

The present invention is defined by a medical device according to claim 1 or 2. Advantageous embodiments of the invention are defined in the dependent claims.

A device embodying the present invention may comprise a flexible surface with nanotubes disposed on the inflatable surface. The device may also be a balloon-type catheter, with nanotubes disposed on a surface of the balloon. These devices and others may be used to deposit material on or transplant material to the surface of an implanted medical device implanted in the body of a patient.

### BRIEF DESCRIPTION OF THE DRAWINGS

FIG. 1 shows enlarged perspective side and sectional views of a balloon with nanotubes disposed on an exposed surface.

FIG. 2 shows enlarged side and sectional views of a device having exterior and interior balloons with nanotubes disposed on an exposed surface of the exterior balloon.

FIG. 3 shows an enlarged side view of a device having an inflatable balloon with nanotubes disposed on a surface positioned in the vessel of a patient.

FIG. 4 shows an enlarged perspective view of a nanotube comprising a piezoelectric material.

FIG. 5 shows a manner in which a device having an inflatable balloon witch nanotubes may be disposed on a surface positioned at a harvest site within the vessel of a patient.

FIG. 6 shows a manner in which a device having an inflatable balloon with nanotubes may be disposed on a surface positioned at a treatment site within the vessel of a patient.

FIG. 7 shows cross sections of a vessel prior to, during, and after the implantation of a stent.

FIG. 8 shows a sectional view of a vessel before, during, and after a device having an inflatable balloon with nanotubes disposed on a surface is used to treat the vessel.

### DETAILED DESCRIPTION

A balloon catheter or other device with a flexible or expandable region comprising nanotubes is provided. The nanotubes may be disposed on the outside of the balloon, such that when the balloon is placed inside a vessel such as a coronary artery and inflated, the nanotubes may contact an exposed surface of the vessel. The nanotubes may then be actuated, for example by vibration, in order to harvest endothelial cells from a healthy region of the vessel. The balloon may then be deflated and repositioned at an intended delivery site, such as the implant site of a stent. When the balloon is expanded at the delivery site, the nanotubes may be actuated such that the harvested endothelial cells are deposited at the intended delivery site.

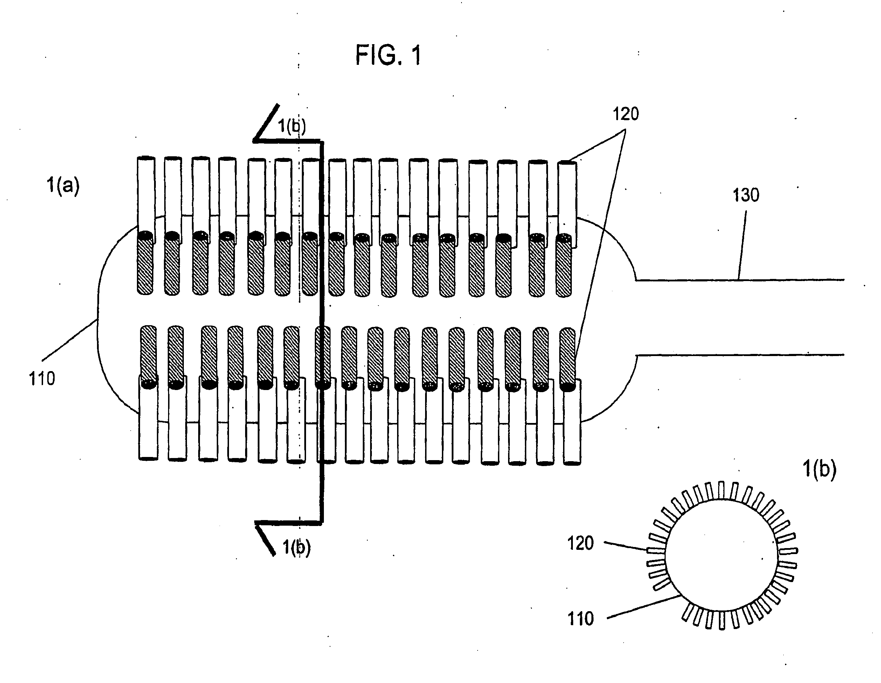

Figure 1 shows a balloon 110 with nanotubes 120 disposed on a surface. Inflatable balloon 110 is disposed on an elongated member 130 suitable for delivering the device to an intended treatment site, such as within the body of a patient. The elongated member 130 may be, for example, a catheter. A plurality of nanotubes 120 is disposed on the outer surface of balloon 110. Nanotubes 120 may be roughly cylindrical, with lengths of approximately 100 micrometers and diameters in the range of about 100 - 4000 nm. The nanotubes may be hollow. They may be fabricated from carbon, ferroelectric materials, oxides, piezoelectric materials, or other appropriate materials. Nanotubes 120 may be in fluid communication with the interior of balloon 110. When the nanotubes 120 are in fluid communication with the interior of the balloon 110, at least some fluids may move freely between the interiors of the nanotubes and the interior of the balloon. For example, the nanotubes 120 may be in fluid communication with the interior of the balloon 110 such that a vacuum applied to the interior of the balloon 110 will propagate through the nanotubes 120, causing material to be drawn into the interior of the nanotubes. The nanotubes may also be partially in fluid communication with the interior of the balloon such that a vacuum may be applied to the interior of the nanotubes, but material drawn into the interiors of the nanotubes may be prohibited from entering the interior of the balloon 110. The nanotubes 120 may be disposed uniformly over the surface of balloon 110. They may be disposed in a grid, or in other suitable arrangements.

A cross-section of balloon 110 is shown in Figure 1(b). A representative sample of nanotubes 120 is shown, with several not displayed for clarity. The nanotubes may be disposed in other arrangements, with more or fewer nanotubes than shown in Figure 1. The nanotubes as shown in the figures described herein may not be shown to scale. Nanotubes 120 may be disposed such that each nanotube is roughly perpendicular to the surface of the balloon when the balloon is inflated as shown in the cross-section of Figure 1. Nanotubes may be disposed in various places on the entire surface of balloon 110, or they may be disposed within particular regions. For some applications, nanotubes 120 may be confined to areas of balloon 110 intended to serve specific functions.

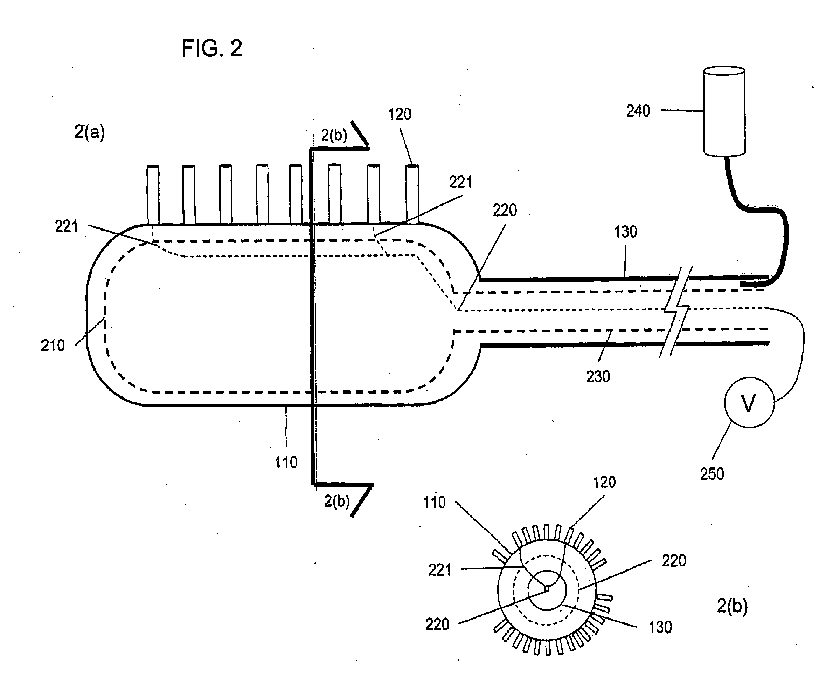

Devices according to the present invention may comprise an interior balloon, an exterior balloon, and nanotubes disposed on the exterior surface of the exterior balloon. Figure 2 shows a side view and a cross-section of a two-balloon device having nanotubes disposed on the surface. Balloon 110 is disposed on elongated member 130. Nanotubes 120 are disposed on the outer surface of balloon 110 as previously described with reference to Figure 1. Nanotubes 120 may comprise a piezoelectric material. Inner balloon 210 is disposed in the interior of balloon 100. The inner balloon 210 may be used to inflate the device, for example by filling it with a fluid such as a saline solution. Inner balloon 210 may be disposed adjacent to and in fluid communication with interior lumen 230 to allow for fluid to be delivered to the interior of balloon 210. The exterior balloon may be used to apply a vacuum to the nanotubes, for example to harvest endothelial cells from a healthy region of a vessel. A vacuum may be applied to the exterior balloon 110 and through the nanotubes 120. The balloon may then be deflated, repositioned at the intended delivery site, and re-inflated. Materials such as previously-harvested endothelial cells may then be deposited by applying pressure to the nanotubes through the exterior balloon.

The nanotubes may comprise a piezoelectric actuator. The device illustrated in Figure 2 may further comprise electrically conductive element 220 disposed within the interior of balloon 110 and elongated member 130. Conductive element 220 may be electrically connected via leads 221 to nanotubes 120. Conductive element 220 may be electrically connected to all or some of the nanotubes, with only representative connections being shown in Figure 2. Wire or other conductive element 220 may be disposed within interior balloon 210 and interior lumen 230 as shown, or it may be disposed within the interior of balloon 110 and elongated member 130, but external to interior balloon 210 and interior lumen 230. Other configurations are possible; for example the conductive element 220 may be disposed on the outside of elongated member 130 or balloon 110. In such embodiments, the nanotubes may be connected to an external voltage source 250, such that when a voltage is applied the nanotubes 120 are deformed or otherwise actuated. For example, the nanotubes 120 may increase in diameter, thus aiding the harvest of material such as healthy endothelial cells. A voltage may also be applied to the nanotubes 120 in order to deposit material such as endothelial cells or a therapeutic agent at a treatment site. For example, a voltage may be applied such that nanotubes 120 increase in diameter, vibrate, or otherwise become active.

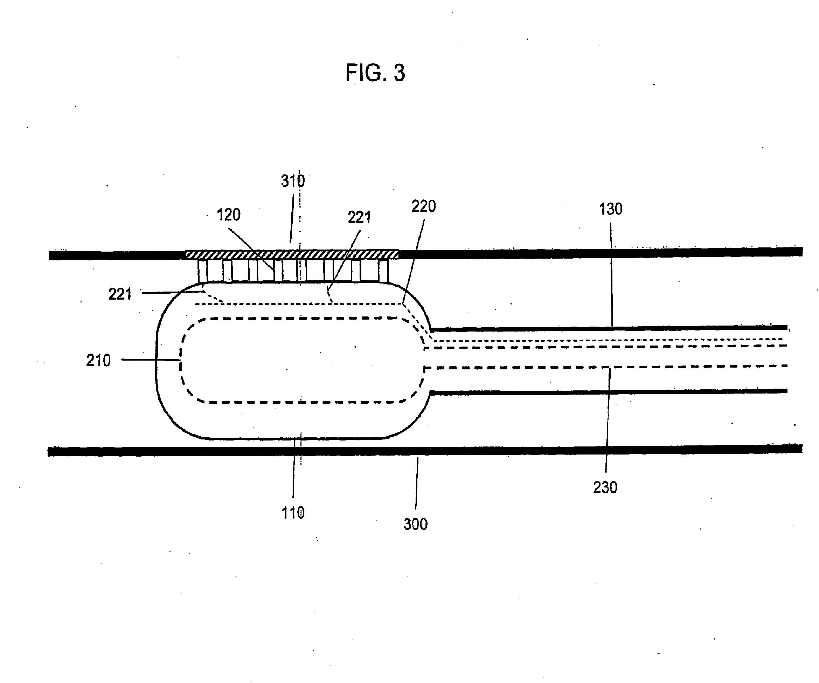

Figure 3 shows an enlarged cross-section of a delivery device positioned within the vessel of a patient. Balloon 110 may be positioned at vessel site 310 within vessel 300 by a practitioner. Balloon 110 may then be inflated such that nanotubes 120 contact site 310. Inner balloon 210 may be used to inflate balloon 110. For example, a fluid such as saline may be inserted into inner balloon 210, such that it expands. Balloon 110 may also be inflated by inserting a fluid into the interior of inner balloon 110.

When balloon 110 is inflated, nanotubes 120 may contact site 310 as shown. Nanotubes 120 may then be actuated by a practitioner in order to harvest material from site 310. For example, if site 310 comprises healthy endothelial cells, nanotubes 120 may be actuated in order to harvest healthy endothelial cells for later transplant at a site within the vessel. Such actuation may comprise applying a vacuum to the interior of balloon 110. When a vacuum is applied, healthy endothelial cells or other material may be removed from site 310 and deposited within nanotubes 120. The nanotubes 120 may comprise a piezoelectric material or be otherwise in communication with a piezoelectric actuator, such that they may be actuated by applying a voltage to electrically conductive element 220. Electrically conductive element 220 is electrically connected 221 to nanotubes 120. Various types and methods of connections are possible, with connections 221 shown as an example. When a voltage is applied to nanotubes 120 via an electrically conductive element 220 and connections 221, the nanotubes 120 may be deformed such that they draw material from a site 310 into the interior of the nanotubes 120.

Balloon 110 may be deflated, for example by applying a vacuum or otherwise removing fluid from the interior of balloon 110, after material is collected. Balloon 110 may then be repositioned, for example at or near the location of an implanted medical device such as a stent, in order to deposit the harvested material.

Site 310 may comprise a site to which previously-harvested cells or other material are to be delivered. For example, it may be the site of an implanted stent, to which harvested healthy endothelial cells are to be delivered. Balloon 110 may be positioned at or near the location of site 310. Balloon 110 may then be inflated as previously described, causing nanotubes 120 to be positioned at, near, or in contact with site 310. Nanotubes 120 may then be actuated such that material disposed within nanotubes 120 is deposited at site 310. For example, if nanotubes 120 were previously used to harvest endothelial cells from a healthy site within vessel 300, these cells may be deposited at site 310. Alternatively, nanotubes 120 may be actuated by, for example, applying pressure via fluid to the interior of balloon 110. When such pressure is applied, healthy endothelial cells or other material may be ejected from the interior of nanotubes 120 and deposited at site 310. Nanotubes 120 may comprise a piezoelectric material, such that they may be actuated by applying a voltage to electrically conductive element 220. Electrically conductive element 220 may be electrically connected 221 to nanotubes 120. It will be understood that various types and methods of connection are possible, with connections 221 shown as an example. When a voltage is applied to nanotubes 120 via electrically conductive element 220 and connections 221, nanotubes 120 may be deformed such that they eject material from within nanotubes 120 to site 310. It may be preferred for balloon 110 to be approximately 20% longer than the intended treatment site 310 in order to insure sufficient material is deposited. For example, when healthy endothelial cells are being transplanted to an implanted stent, it may be desirable for cells to cover not only the stent but also the areas around or near the stent in order to encourage endothelialization of the entire area.

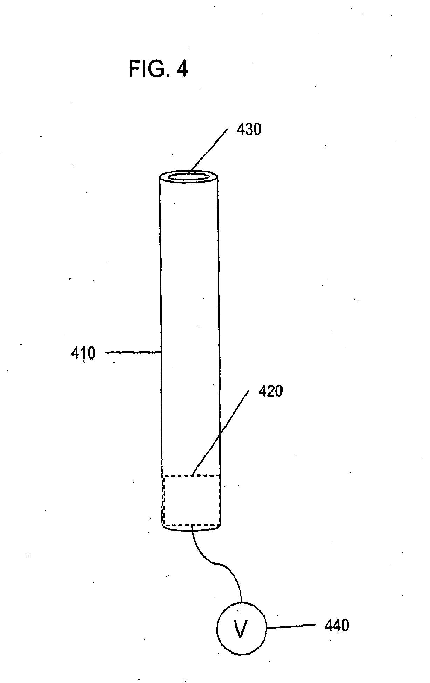

Figure 4 shows an enlarged view of an example of an actuator in accord with the present invention. Nanotube 410 comprises piezoelectric material 420 connected to external voltage source 440. When a voltage is applied, piezoelectric material 420 may be deformed such that material is drawn through nanotube opening 430 into the interior of nanotube 410. For example, piezoelectric material 420 may compress along the longitudinal axis of nanotube 410 when a voltage is applied, causing a vacuum within nanotube 410. Material adjacent to nanotube opening 430 may thus be drawn in to the nanotube. Similarly, the entirety of nanotube 410 may comprise a piezoelectric material, such that application of a voltage causes nanotube 410 to be deformed. For example, a voltage may be applied that causes nanotube 410 to increase in diameter, resulting in a decrease in pressure within nanotube 410. Other configurations are possible.

Piezoelectric material 420 may also be used to eject material from the nanotubes. Nanotube 410 comprises piezoelectric material 420 connected to external voltage source 440. When a voltage is applied, piezoelectric material 420 may be deformed such that material is ejected from nanotube opening 430. For example, piezoelectric material 420 may expand along the longitudinal axis of nanotube 410 when a voltage is applied, causing outward pressure within nanotube 410. Material within nanotube 410 may thereby be ejected. Similarly, the entirety of nanotube 410 may comprise a piezoelectric material, such that application of a voltage causes nanotube 410 to be deformed. For example, application of a voltage may cause nanotube 410 to decrease in diameter, resulting in an increased pressure within nanotube 410. Other configurations of piezoelectric material are possible.

Devices according to the present invention are used to cause material such as endothelial cells to be drawn into the nanotubes. A therapeutic agent or other substance may be disposed on or within the nanotubes. The device may then be used to deliver the substance to an intended delivery site within a vessel. For example, a growth factor such as Vascular Endothelial Growth Factor (VEGF) may be disposed on the nanotubes, after which the balloon may be placed at the intended delivery site as previously described. The balloon may then be inflated and the nanotubes actuated so as to deposit the growth factor or other substance at the delivery site. As an example, the device may deposit VEGF on the surface of a stent to encourage endothelialization of the stent.

Referring to Figure 3, nanotubes 120 may examplarily be used to deliver a therapeutic agent or other material to site 310. Prior to positioning balloon 110 at or near site 310, therapeutic agent may be placed in nanotubes 120. The therapeutic agent may be placed within nanotubes 120 by using the methods previously described. For example, nanotubes 120 could be actuated while disposed adjacent to or within a therapeutic agent, causing.therapeutic agent to be drawn in to the nanotubes. Balloon 110 may then be placed at or near treatment site 310 and inflated as previously described. Nanotubes 120 may then be actuated, causing the material disposed within the nanotubes to be deposited at site 310. As an example, site 310 may comprise an implanted stent or other medical device. A therapeutic agent, such as VEGF, may be placed on the nanotubes and delivered to the implanted stent as described previously. Such a procedure may allow for sensitive therapeutic agents or other materials to be delivered to an implanted stent without requiring the material to be placed on the stent prior to implantation.

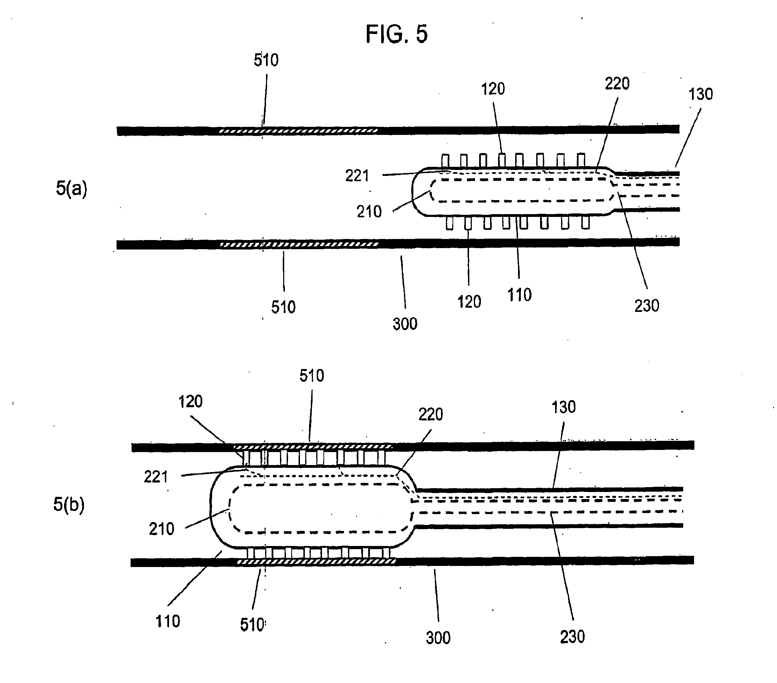

Devices according to the present invention may also be used to transplant material from one site within a vessel to another site within a vessel. Figure 5 shows a balloon catheter having nanotubes 120 on the surface of the balloon 110 disposed near a harvest site 510 within the vessel of a patient 300. Balloon 110 is disposed at the distal end of catheter 130. The harvest site 510 may comprise, for example, healthy endothelial cells. Referring to Figure 5(a), the balloon 110 may initially be in a deflated first position, such that catheter 130 may be moved within the vessel 300. The device may be positioned by a practitioner such that the balloon 110 is adjacent to the site 510 from which material is to be harvested. Figure 5(b) shows the balloon 110 positioned in a location within vessel 300 such that endothelial cells may be harvested from harvest site 510. The balloon may then be inflated such that the nanotubes 120 are near or in contact with the harvest site 510. The balloon may be inflated by a variety of methods, for example by inflating balloon 110 or inner balloon 210 with a fluid as previously described. A practitioner may actuate the nanotubes 120 such that cells are removed from the harvest site 510 of the vessel. For example, the nanotubes may be actuated piezoelectrically by applying a voltage to conductive elements 220 and 221, or a vacuum may be applied to the interior of balloon 110 as previously described. As the nanotubes are actuated, cells may be harvested from the harvest site 510. The balloon may then be deflated as shown in Figure 5(a) and repositioned at an intended treatment site (not shown).

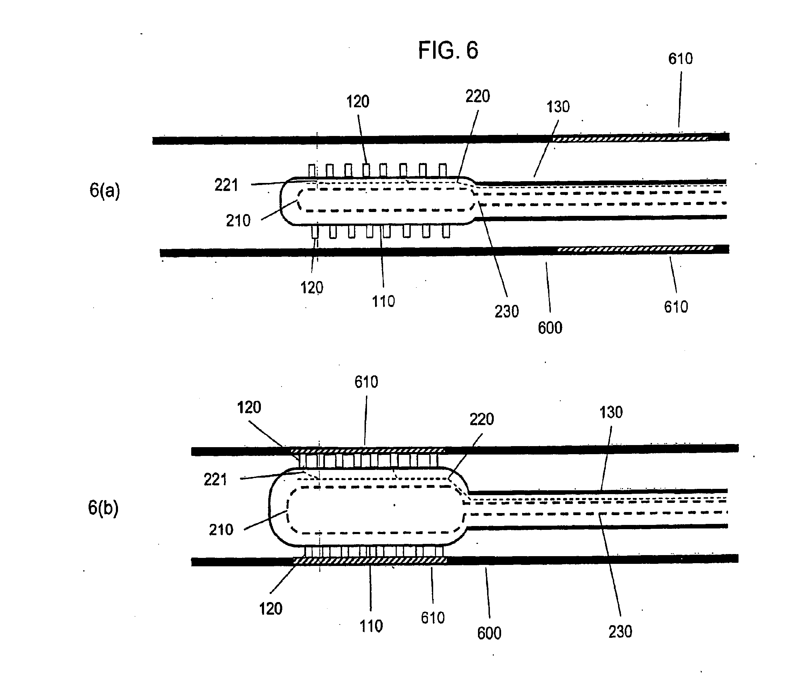

Figure 6 shows the balloon catheter illustrated in Figure 5, positioned in vessel 600 near an intended treatment site 610. Vessel 600 may be the same vessel as vessel 300 shown in Figure 5, or it may be a different vessel. The intended treatment site 610 may comprise, for example, a stent that has been implanted into vessel 600. As shown in Figure 6(a), the balloon 110 may be deflated to allow a practitioner to position the balloon near delivery site 610. Once the balloon is positioned, it may be inflated as shown in Figure 6(b) such that the nanotubes 120 may be positioned adjacent to or in contact with delivery site 610. The balloon 110 may be inflated using a variety of methods as previously described. Once the nanotubes 120 are positioned as desired, a practitioner may actuate them such that material disposed within the nanotubes is ejected onto treatment site 610. The material may comprise, for example, endothelial cells harvested as described with respect to Figure 5 or as an example a therapeutic agent such as VEGF. The nanotubes 120 may be actuated by a variety of methods as previously described, including piezoelectrically or by applying fluid pressure to the interior of balloon 110.

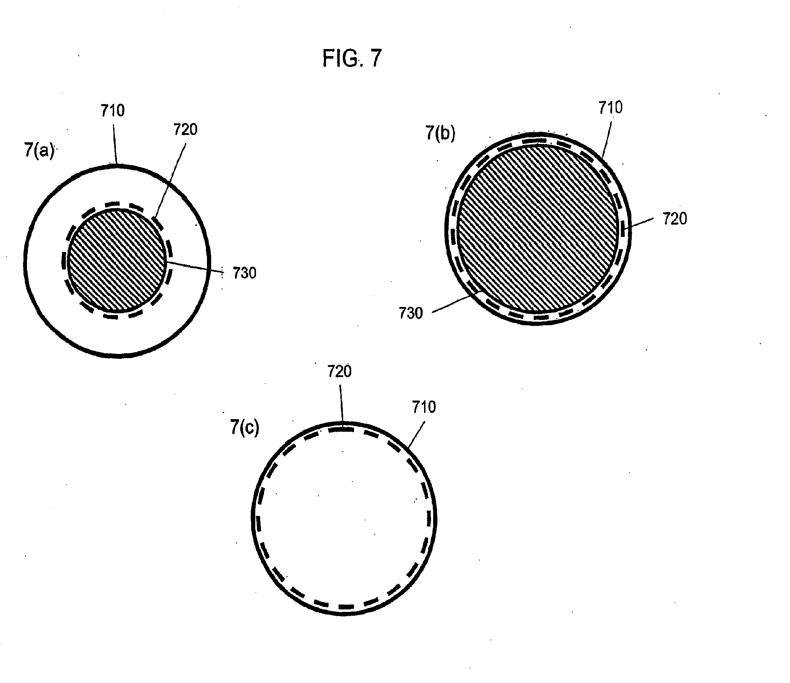

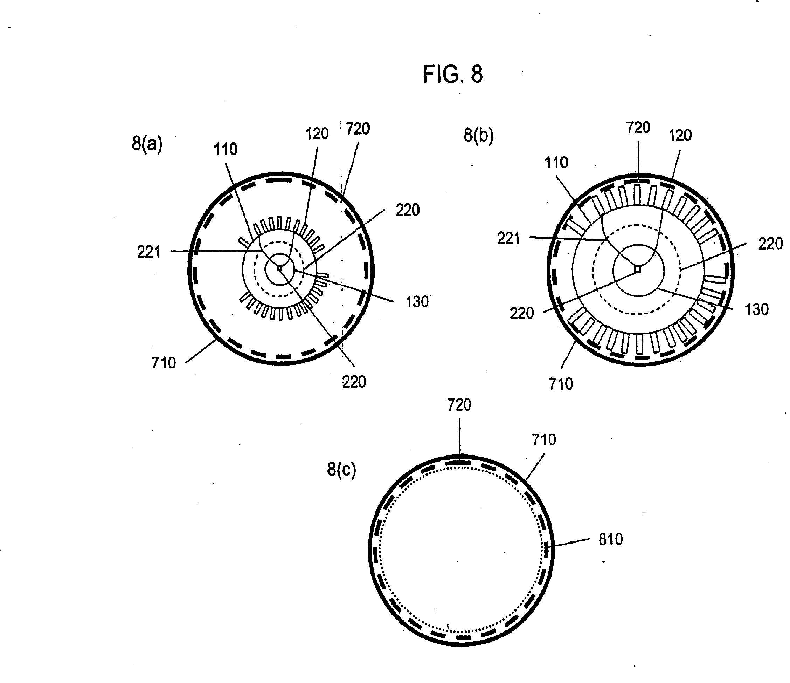

A medical device such as a stent may be implanted within the vessel of a patient. Figure 7 shows a cross section of a vessel with an implanted stent. As illustrated in Figure 7(a), a balloon catheter 730 may be inserted into a vessel 710 of a patient. A stent 720 may be disposed on the outside of the balloon. When the balloon 730 is inflated as shown in Figure 7(b), the stent 720 may be placed on the surface of the vessel 710. Such an arrangement may be used, for example, as a treatment to prevent closure of the vessel. When the balloon catheter is removed as shown in Figure 7(c), the stent may remain in order to continue providing treatment to the vessel. In order to prevent adverse effects such as stent thrombosis, it may be preferable to administer a therapeutic agent or endothelial cells to the location of the stent. Figure 8(a) shows a device comprising an inflatable balloon 110 with nanotubes 120, as previously described, disposed near the site of stent 720. A representative illustration of nanotubes is shown; more or fewer nanotubes may be used. The balloon 110 may be physically connected to a device 130 such as a catheter, endoscope, or thorascope to allow for placement near the stent 720. The device shown in Figure 8(a), has previously been used to collect endothelial cells from within a vessel as previously described. When balloon 110 is inflated as shown in Figure 8(b), the nanotubes 120 may be disposed near or in contact with the stent 720. A practitioner may then actuate the nanotubes 120, for example by applying a voltage using conductive element 220 and connections 221, or using other methods as previously described. Material 810 within or on the nanotubes 120 may be deposited on or around the stent 720 as illustrated in Figure 8(c). For example, a therapeutic agent or endothelial cells may be deposited on the surface of stent 720, in the regions between the stent 720 and the vessel surface 710, or both. The balloon 110 may be deflated and withdrawn after material has been deposited.

As used herein, the "proximal" end of a device or portion of a device refers to the end closest to a practitioner operating the device. Similarly, the "distal" end of a device or portion of a device refers to the end farthest from the operator of the device.

The term "therapeutic agent" as used throughout includes one or more "therapeutic drugs" or "genetic material." The term "therapeutic agent" used herein includes pharmaceutically active compounds, nucleic acids with and without carrier vectors such as lipids, compacting agents (such as histones), virus (such as adenovirus, adenoassociated virus, retrovirus, lentivirus and a-virus), polymers, hyaluronic acid, proteins, cells and the like, with or without targeting sequences. The therapeutics administered in accordance with the invention includes the therapeutic agent(s) and solutions thereof.

The therapeutic agent may be any pharmaceutically acceptable agent such as a non-genetic therapeutic agent, a biomolecule, a small molecule, or cells.

Exemplary non-genetic therapeutic agents include anti-thrombogenic agents such heparin, heparin derivatives, prostaglandin (including micellar prostaglandin E1), urokinase, and PPack (dextrophenylalanine proline arginine chloromethylketone); anti-proliferative agents such as enoxaprin, angiopeptin, sirolimus (rapamycin), tacrolimus, everolimus, zotarolimus, monoclonal antibodies capable of blocking smooth muscle cell proliferation, hirudin, and acetylsalicylic acid; anti-inflammatory agents such as dexamethasone, rosiglitazone, prednisolone, corticosterone, budesonide, estrogen, estrodiol, sulfasalazine, acetylsalicylic acid, mycophenolic acid, and mesalamine; anti-neoplastic/anti-proliferative/anti-mitotic agents such as paclitaxel, epothilone, cladribine, 5-fluorouracil, methotrexate, doxorubicin, daunorubicin, cyclosporine, cisplatin, vinblastine, vincristine, epothilones, endostatin, trapidil, halofuginone, and angiostatin; anti-cancer agents such as antisense inhibitors of c-myc oncogene; antimicrobial agents such as triclosan, cephalosporins, aminoglycosides, nitrofurantoin, silver ions, compounds, or salts; biofilm synthesis inhibitors such as non-steroidal anti-inflammatory agents and chelating agents such as ethylenediaminetetraacetic acid, O,O'-bis (2-aminoethyl)ethyleneglycol-N,N,N',N'-tetraacetic acid and mixtures thereof; antibiotics such as gentamycin, rifampin, minocyclin, and ciprofolxacin; antibodies including chimeric antibodies and antibody fragments; anesthetic agents such as lidocaine, bupivacaine, and ropivacaine; nitric oxide; nitric oxide (NO) donors such as linsidomine, molsidomine, L-arginine, NO-carbohydrate adducts, polymeric or oligomeric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, warfarin sodium, Dicumarol, aspirin, prostaglandin inhibitors, platelet aggregation inhibitors such as cilostazol and tick antiplatelet factors; vascular cell growth promotors such as growth factors, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vascoactive mechanisms; inhibitors of heat shock proteins such as geldanamycin; angiotensin converting enzyme (ACE) inhibitors; beta-blockers; bAR kinase (bARKct) inhibitors; phospholamban inhibitors; protein-bound particle drugs such as ABRAXANE™; and any combinations and prodrugs of the above.

Exemplary biomolecules include peptides, polypeptides and proteins; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Nucleic acids may be incorporated into delivery systems such as, for example, vectors (including viral vectors), plasmids or liposomes.

Non-limiting examples of proteins include serca-2 protein, monocyte chemoattractant proteins ("MCP-1") and bone morphogenic proteins ("BMP's"), such as, for example, BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15. Preferred BMPS are any of BMP-2, BMP-3, BMP-4, BMP-5, BMP-6, and BMP-7. These BMPs can be provided as homdimers, heterodimers, or combinations thereof, alone or together with other molecules. Alternatively, or in addition, molecules capable of inducing an upstream or downstream effect of a BMP can be provided. Such molecules include any of the "hedghog" proteins, or the DNA's encoding them. Non-limiting examples of genes include survival genes that protect against cell death, such as anti-apoptotic Bcl-2 family factors and Akt kinase; serca 2 gene; and combinations thereof. Non-limiting examples of angiogenic factors include acidic and basic fibroblast growth factors, vascular endothelial growth factor, epidermal growth factor, transforming growth factor ÿ and ÿ, platelet-derived endothelial growth factor, platelet-derived growth factor, tumor necrosis factor ÿ, hepatocyte growth factor, and insulin like growth factor. A non-limiting example of a cell cycle inhibitor is a cathespin D (CD) inhibitor. Non-limiting examples of anti-restenosis agents include p15, p16, p18, p19, p21, p27, p53, p57, Rb, nFkB and E2F decoys, thymidine kinase ("TK") and combinations thereof and other agents useful for interfering with cell proliferation.

Exemplary small molecules include hormones, nucleotides, amino acids, sugars, and lipids and compounds have a molecular weight of less than 100kD.

Exemplary cells include stem cells, progenitor cells, endothelial cells, adult cardiomyocytes, and smooth muscle cells. Cells can be of human origin (autologous or allogenic) or from an animal source (xenogenic), or genetically engineered. Non-limiting examples of cells include side population (SP) cells, lineage negative (Lin-) cells including Lin-CD34-, Lin-CD34+, Lin-cKit+, mesenchymal stem cells including mesenchymal stem cells with 5-aza, cord blood cells, cardiac or other tissue derived stem cells, whole bone marrow, bone marrow mononuclear cells, endothelial progenitor cells, skeletal myoblasts or satellite cells, muscle derived cells, go cells, endothelial cells, adult cardiomyocytes, fibroblasts, smooth muscle cells, adult cardiac fibroblasts + 5-aza, genetically modified cells, tissue engineered grafts, MyoD scar fibroblasts, pacing cells, embryonic stem cell clones, embryonic stem cells, fetal or neonatal cells, immunologically masked cells, and teratoma derived cells.

Any of the therapeutic agents may be combined to the extent such combination is biologically compatible.

Any of the above mentioned therapeutic agents may be incorporated into a polymeric coating on a medical device or applied onto a polymeric coating on a medical device such as, for example, a stent. Such coated devices may be used with, in addition to, or in conjunction with devices according to the present invention. The polymers of the polymeric coatings may be biodegradable or non-biodegradable. Non-limiting examples of suitable non-biodegradable polymers include polystrene; polyisobutylene copolymers, styrene-isobutylene block copolymers such as styrene-isobutylene-styrene tri-block copolymers (SIBS) and other block copolymers such as styrene-ethylene/butylene-styrene (SEBS); polyvinylpyrrolidone including cross-linked polyvinylpyrrolidone; polyvinyl alcohols, copolymers of vinyl monomers such as EVA; polyvinyl ethers; polyvinyl aromatics; polyethylene oxides; polyesters including polyethylene terephthalate; polyamides; polyacrylamides; polyethers including polyether sulfone; polyalkylenes including polypropylene, polyethylene and high molecular weight polyethylene; polyurethanes; polycarbonates, silicones; siloxane polymers; cellulosic polymers such as cellulose acetate; polymer dispersions such as polyurethane dispersions (BAYHDROL®); squalene emulsions; and mixtures and copolymers of any of the foregoing.

Non-limiting examples of suitable biodegradable polymers include polycarboxylic acid, polyanhydrides including maleic anhydride polymers; polyorthoesters; poly-amino acids; polyethylene oxide; polyphosphazenes; polylactic acid, polyglycolic acid and copolymers and mixtures thereof such as poly(L-lactic acid) (PLLA), poly(D,L,-Iactide), poly(lactic acid-co-glycolic acid), 50/50 (DL-lactide-co-glycolide); polydioxanone; polypropylene fumarate; polydepsipeptides; polycaprolactone and co-polymers and mixtures thereof such as pply(D,L-lactide-co-caprolactone) and polycaprolactone co-butylacrylate; polyhydroxybutyrate valerate and blends; polycarbonates such as tyrosine-derived polycarbonates and arylates, polyiminocarbonates, and polydimethyltrimethylcarbonates; cyanoacrylate; calcium phosphates; polyglycosaminoglycans; macromolecules such as polysaccharides (including hyaluronic acid; cellulose, and hydroxypropylmethyl cellulose; gelatin; starches; dextrans; alginates and derivatives thereof), proteins and polypeptides; and mixtures and copolymers of any of the foregoing. The biodegradable polymer may also be a surface erodable polymer such as polyhydroxybutyrate and its copolymers, polycaprolactone, polyanhydrides (both crystalline and amorphous), maleic anhydride copolymers, and zinc-calcium phosphate.

Such coatings may be formed by any method known to one in the art. For example, an initial polymer/solvent mixture can be formed and then the therapeutic agent added to the polymer/solvent mixture. Alternatively, the polymer, solvent, and therapeutic agent can be added simultaneously to form the mixture. The polymer/solvent/therapeutic agent mixture may be a dispersion, suspension or a solution. The therapeutic agent may also be mixed with the polymer in the absence of a solvent. The therapeutic agent may be dissolved in the polymer/solvent mixture or in the polymer to be in a true solution with the mixture or polymer, dispersed into fine or micronized particles in the mixture or polymer, suspended in the mixture or polymer based on its solubility profile, or combined with micelle-forming compounds such as surfactants or adsorbed onto small carrier particles to create a suspension in the mixture or polymer. The coating may comprise multiple polymers and/or multiple therapeutic agents.

The coating can be applied to the medical device by various methods including dipping, spraying, rolling, brushing, electrostatic plating or spinning, vapor deposition, air spraying including atomized spray coating, and spray coating using an ultrasonic nozzle:

The coating is typically from about 1 to about 50 microns thick. In the case of balloon catheters, the thickness is preferably from about 1 to about 10 microns, and more preferably from about 2 to about 5 microns. Very thin polymer coatings, such as about 0.2-0.3 microns and much thicker coatings, such as more than 10 microns, are also possible. It is also within the scope of the present invention to apply multiple layers of polymer coatings onto a medical device used with, in addition to, or in conjunction with the present invention. Such multiple layers may contain the same or different therapeutic agents and/or the same or different polymers. Methods of choosing the type, thickness and other properties of the polymer and/or therapeutic agent to create different release kinetics are well known to one in the art.

The medical device may also contain a radio-opacifying agent within its structure to facilitate viewing the medical device during insertion and at any point while the device is implanted. Non-limiting examples of radio-opacifying agents are bismuth subcarbonate, bismuth oxychloride, bismuth trioxide, barium sulfate, tungsten, and mixtures thereof.

Non-limiting examples of medical devices according to the present invention include catheters, guide wires, balloons, filters (e.g., vena cava filters), stents, stent grafts, vascular grafts, intraluminal paving systems, implants and other devices used in connection with drug-loaded polymer coatings. Such medical devices may be implanted or otherwise utilized in body lumina and organs such as the coronary vasculature, esophagus, trachea, colon, biliary tract, urinary tract, prostate, brain, lung, liver, heart, skeletal muscle, kidney, bladder, intestines, stomach, pancreas, ovary, cartilage, eye, bone, and the like.

## Claims

1. A medical device comprising:
an expandable surface (110), the expandable surface sized to fit within an organic vessel, the expandable surface expandable from a first position to a second position; and
a plurality of nanotubes (120, 410) disposed on the expandable surface;
**characterized in that**
at least one of the nanotubes (120, 410) is at least partially in fluid communication with an interior volume defined by the expandable surface such that a vacuum applied to the interior volume of the expandable surface will propagate through the at least one nanotube causing material such as endothelial cells to be drawn into the interior of the nanotubes.

2. A medical device comprising:
an expandable surface (110), the expandable surface sized to fit within an organic vessel, the expandable surface expandable from a first position to a second position; and
a plurality of nanotubes (120, 410) disposed on the expandable surface;
**characterized in that**
at least one of the nanotubes comprises a piezoelectric material, wherein when voltage is applied to the piezoelectric material the nanotubes are deformed thereby causing material such as endothelial cells to be drawn into the interior of the nanotubes.

3. The medical device of claim 1 or claim 2, wherein at least one nanotube (120, 410) from the plurality of nanotubes is approximately perpendicular to the exterior of the expandable surface (110).

4. The medical device of any of claims 1 to 3, wherein the expandable surface is an outer surface of a first inflatable member (110).

5. The device of any of claims 1 to 4, further comprising a second inflatable membrane (210) defining an interior volume disposed within an interior volume defined by the first inflatable membrane (110).

6. The device of claim 5, wherein at least one of the plurality of nanotubes (120, 410) is in fluid communication with an interior volume defined by the first inflatable membrane (110), and the interior of the first inflatable membrane is not in fluid communication with the interior of the second inflatable membrane (210).

7. The medical device of any of claims 1 to 6, further comprising:
a first inflatable balloon (110) defining an interior volume; and
an elongated member (130) capable of positioning the first inflatable balloon at a treatment site within the body of a patient;
wherein the expandable surface is an outer surface of the first inflatable balloon (110).

## Patentansprüche

1. Eine medizinische Vorrichtung, umfassend:
eine ausdehnbare Oberfläche (110), wobei die ausdehnbare Oberfläche dimensioniert ist, um in ein organisches Gefäß zu passen, wobei die ausdehnbare Oberfläche von einer ersten Position in eine zweite Position ausdehnbar ist; und
eine Vielzahl von Nano-Röhren (120, 410), die auf der ausdehnbaren Oberfläche vorgesehen sind;
**dadurch gekennzeichnet, dass**
zumindest eine der Nano-Röhren (120, 410) zumindest teilweise in flüssiger Kommunikation mit einem Innenvolumen steht, welches durch die ausdehnbare Oberfläche so definiert ist, dass ein Vakuum, welches auf das Innenvolumen der ausdehnbaren Oberfläche aufgebracht wird, durch die zumindest eine Nano-Röhre hindurch übertragen wird, was dazu führt, dass Material, wie endothelische Zellen, in das Innere der Nano-Röhren gezogen wird.

2. Medizinische Vorrichtung, umfassend:
eine ausdehnbare Oberfläche (110), wobei die ausdehnbare Oberfläche dimensioniert ist, um in ein organisches Gefäß zu passen, wobei die ausdehnbare Oberfläche von einer ersten Position in eine zweite Position ausdehnbar ist; und
eine Vielzahl von Nano-Röhren (120, 410), die auf der ausdehnbaren Oberfläche angeordnet sind;
**dadurch gekennzeichnet, dass**
zumindest eine der Röhren ein piezoelektrisches Material umfasst, wobei, wenn eine Spannung auf das piezoelektrische Material aufgebracht wird, die Nano-Röhren deformiert werden, was dazu führt, dass Material, wie endothelische Zellen, in das Innere der Nano-Röhren gezogen wird.

3. Medizinische Vorrichtung nach Anspruch 1 oder Anspruch 2, wobei zumindest eine Nano-Röhre (120, 410) aus der Vielzahl von Nano-Röhren ungefähr senkrecht zu dem Äußeren der ausdehnbaren Oberfläche (110) ist.

4. Medizinische Vorrichtung nach irgendeinem der Ansprüche 1 bis 3, wobei die ausdehnbare Oberfläche eine äußere Oberfläche eines ersten aufblasbaren Bauteils (110) ist.

5. Vorrichtung nach irgendeinem der Ansprüche 1 bis 4, weiterhin eine zweite aufblasbare Membran (210) umfassend, die ein Innenvolumen definiert, welches in einem Innenvolumen angeordnet ist, welches durch das erste aufblasbare Bauteil (110) definiert ist.

6. Vorrichtung nach Anspruch 5, wobei zumindest eine aus der Vielzahl der Nano-Röhren (120, 410) in flüssiger Kommunikation mit einem Innenvolumen steht, welches durch das erste aufblasbare Bauteil (110) definiert ist, und das Innere der ersten aufblasbaren Membran nicht in flüssiger Kommunikation mit dem Inneren der zweiten aufblasbaren Membran (210) steht.

7. Medizinische Vorrichtung nach irgendeinem der Ansprüche 1 bis 6, weiterhin umfassend:
einen ersten aufblasbaren Ballon (110), der ein Innenvolumen definiert; und
ein gestrecktes Bauteil (130), welches in der Lage ist, den ersten aufblasbaren Ballon in einem Behandlungsbereich innerhalb des Körpers eines Patienten zu positionieren;
wobei die ausdehnbare Oberfläche eine äußere Oberfläche des ersten aufblasbaren Ballons (110) ist.

## Revendications

1. Un dispositif médical comprenant :
une surface expansible (110), la surface expansible étant dimensionnée pour s'adapter à l'intérieur d'un vaisseau organique, la surface expansible pouvant s'expanser d'une première position à une seconde position ; et
une pluralité de nanotubes (120, 410) disposés sur la surface expansible ;
**caractérisé en ce que**
au moins l'un des nanotubes (120, 410) est au moins partiellement en communication de fluide avec un volume intérieur défini par la surface expansible de sorte qu'une aspiration appliquée au volume intérieur de la surface expansible se propage au travers du au moins un nanotube faisant en sorte que des matières telles que des cellules endothéliales soient aspirées vers l'intérieur des nanotubes.

2. Un dispositif médical comprenant :
une surface expansible (110), la surface expansible étant dimensionnée pour s'adapter à l'intérieur d'un vaisseau organique, la surface expansible pouvant s'expanser d'une première position à une seconde position ; et
une pluralité de nanotubes (120, 410) disposés sur la surface expansible ;
**caractérisé en ce que**
au moins l'un des nanotubes comprend un matériau piézoélectrique, les nanotubes étant déformés lorsqu'une tension est appliquée au matériau piézoélectrique, faisant en sorte que des matières telles que des cellules endothéliales soient aspirées vers l'intérieur des nanotubes.

3. Le dispositif médical de la revendication 1 ou de la revendication 2, dans lequel au moins un nanotube (120, 410) de la pluralité de nanotubes est approximativement perpendiculaire à l'extérieur de la surface expansible (110).

4. Le dispositif médical de l'une des revendications 1 à 3, dans lequel la surface expansible est une surface extérieure d'un premier organe gonflable (110).

5. Le dispositif de l'une des revendications 1 à 4, comprenant en outre une seconde membrane gonflable (210) définissant un volume intérieur disposé à l'intérieur d'un volume intérieur défini par la première membrane gonflable (110).

6. Le dispositif de la revendication 5, dans lequel au moins un de la pluralité de nanotubes (120, 410) est en communication de fluide avec un volume intérieur défini par la première membrane gonflable (110), et l'intérieur de la première membrane gonflable n'est pas en communication de fluide avec l'intérieur de la seconde membrane gonflable (210).

7. Le dispositif médical de l'une des revendications 1 à 6, comprenant en outre :
un premier ballonnet gonflable (110) définissant un volume intérieur ; et
un organe allongé (130) capable de positionner le premier ballonnet gonflable en un site de traitement à l'intérieur du corps d'un patient ;
la surface expansible étant une surface extérieure du premier ballonnet gonflable (110).
